(19) Europäisches Patentamt
European Patent Office
Office européen des brevets

(11) Veröffentlichungsnummer: **0 043 974**
**B1**

# (12) EUROPÄISCHE PATENTSCHRIFT

(45) Veröffentlichungstag der Patentschrift: **20.06.84**

(51) Int. Cl.³: **A 61 K 31/785**

(21) Anmeldenummer: **81105042.6**

(22) Anmeldetag: **30.06.81**

## (54) Antitumoral wirkende Mittel.

(30) Priorität: **12.07.80 DE 3026575**

(43) Veröffentlichungstag der Anmeldung: **20.01.82 Patentblatt 82/3**

(45) Bekanntmachung des Hinweises auf die Patenterteilung: **20.06.84 Patentblatt 84/25**

(84) Benannte Vertragsstaaten: **AT BE CH DE FR GB IT LI NL SE**

(56) Entgegenhaltungen:
**L.G. DONARUMA: "Polymeric drugs", 1978, Seiten 240, 245 Academic Press New York, U.S.A.**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Wolf, Gerhard Dieter, Dr., Wilhelm-Busch-Strasse 29, D-4047 Dormagen 5 (DE)**
Erfinder: **Bömer, Bruno, Dr., Gustav-Freytag-Strasse 2, D-5090 Leverkusen 1 (DE)**
Erfinder: **Bierling, Robert, Dr., Merlinweg 18 b, D-5600 Wuppertal 1 (DE)**

**EP 0 043 974 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von an sich bekannten wasserlöslichen Additionsprodukten aus einem Polyetherdiol und einem Diisocyanat als antitumoral wirkende Mittel.

Es ist schon bekannt geworden, dass copolymere, komplexe Cu(II)- und Co(II)-Salze der Ethylenmaleinsäure gegen das Walker-Sarkom wirksam sind („J. Med. Chem.", *12* [1969], 1180).

Polyoxyethylensorbitanmonooleat (Tween 80) ist schon für die Immunisierung gegen den hyperdiploiden Ehrlich-Tumor verwendet worden („Experientia", *29* [1973], 710).

Weiterhin wurden Polykationen verschiedenen Typs, z.B. Polyamidamine, Poly-N-morpholinoethylacrylamid und N-Oxid-Polymere auf Hemmung der Metastasierung geprüft mit dem Ergebnis, dass nur die Tumorzellaussaat, nicht aber das Metastasenwachstum *in situ* bzw. Lymphknotenmetastasen beeinflusst werden konnten („J. Med. Chem.", *16* [1973], 496).

Ferner wurde die Wirksamkeit von Polymeren mit Carboxylgruppen gegen das Sarkom 180 in Abhängigkeit von dem Molekulargewicht, der Ladungsdichte sowie der Metallbindungsfähigkeit der Carboxylgruppen beschrieben („Dissertation Abstr. Intern. B" *33* [1973], 5745).

Polyaninen, z.B. Polyamoniumacrylat, Acrylsäureacrylamidcopolymere sowie Copolymere aus Ethylen- und Maleinsäureanhydrid sollen im Zusammenhang mit ihrer Antitumorwirkung einen heparinähnlichen Effekt, daneben eine Virushemmung besitzen und ausserdem die Immunreaktionen steigern („J. Med. Chem.", *17* [1974], 1335).

Aus allen diesen Arbeiten geht hervor, dass die Antitumorwirkung der bisher untersuchten Polymere an den verwendeten experimentellen Tumoren oft nur an der unteren Grenze der Signifikanz liegt und verschiedentlich nur auf prophylaktische oder adjuvantive Effekte beschränkt ist. Nachteilig erscheint ausserdem, dass die zitierten Untersuchungen vielfach an allogenen, zur Spontanregression neigenden Mäusetumoren und nicht systematisch und unter kliniknahen Versuchsanordnungen durchgeführt wurden. Meist fehlen Angaben zur Toxizität der Präparate, obwohl die Applikation hoher Dosierungen von Substanzen mit einem Molekulargewicht von über 30000 eine mangelhafte Ausscheidung bzw. Speicherung in den Geweben vermuten lässt.

Ein Blockcopolymerisat aus Polyoxypropylen und Polyoxyethylen (Pluronic F 68) erwies sich als wirksam gegen den Metastasenangang des Walker 256 Ascites Tumors, wahrscheinlich durch Beeinflussung der Blutgerinnungsfähigkeit („Cancer", *29* [1972], 171). Diese Präparate sind bekanntermassen hoch wirksame Emulgatoren und sind aus diesem Grunde nicht sehr gut verträglich insbesondere bei parenteraler Applikation.

W. Regelson *et al.* („Nature", 186 [1960], 778-780) untersuchten die Antitumorwirkung synthetischer Polyelektrolyte wie Polyacrylsäure, Polymethacrylsäure und hydrolysierten bzw. aminolysierten Ethylen/Maleinsäureanhydrid-Copolymerisaten. Sie fanden durch Wirkungsvergleich der Dicarbonsäure-, der Amidcarbonsäure und der Diamidform von Ethylen/Maleinsäureanhydrid-Copolymerisaten, dass für eine signifikante Tumorinhibierung mindestens eine ionisierbare Carboxylgruppe erforderlich ist. Versuche dieser Autoren mit Polyacrylamiden ergaben in hohen Dosen (800 mg/kg, Molekulargewicht 60-70 000 und 400 mg/kg, Molekulargewicht 120000) eine negative bzw. eine nicht signifikante positive Tumorhemmwirkung.

Ebenfalls wurden antitumoral wirkende Mittel beschrieben, die durch einen Gehalt an mindestens einem wasserlöslichen Homo- oder Copolymerisat, das das 1,3-Dihydroxy-2-methylenpropan und/oder dessen Derivate enthält, charakterisiert sind (DOS Nr. 2705189). Ähnliche Präparate mit ähnlicher Wirkung sind wasserlösliche Homo- oder Copolymerisate, die 3,4-Dihydroxybuten-1 oder Hydroxyalkyl(meth)acrylat sowie deren Derivate oder auch Derivate des Allylalkohols polymerisiert oder copolymerisiert enthalten (DOS Nr. 2740082).

Es wurde nun gefunden, dass die an sich bekannten wasserlöslichen Additionsprodukte aus einem Polyetherdiol und einem Diisocyanat starke antitumorale Eigenschaften besitzen.

Überraschenderweise zeigen diese Substanzen im Molekulargewichtsbereich von 1000 bis 30000 signifikante kurative Wirkungen an soliden Tumoren syngener Systeme in einem breiten Dosisbereich von 0,5 bis 500 mg/kg, vorzugsweise 5 bis 250 mg/kg, unter kliniknahen Versuchsanordnungen und Applikationsarten. Die therapeutische Breite der Substanzen erweist sich dabei also ungewöhnlich gross ($LD_{50}$, i.v.: >2500 mg/kg, therapeutische Dosis: 0,5 bis 500 mg/kg).

Die erfindungsgemässen Mittel stellen somit eine wichtige Bereicherung der Therapie dar.

Die Wirkstoffe für die erfindungsgemässen Mittel sind wasserlösliche Oligo- bzw. Polyurethane der allgemeinen Formel

$$B\{Q-O-CO-NH-R-NH-CO\}_mQ-O-B$$

in welcher Q einen gegebenenfalls ionische Gruppen enthaltenden Polyetherrest, bevorzugt einen Rest der Formel

$$\left(O-\underset{\displaystyle |}{\overset{\displaystyle A}{C}}H-CH_2\right)_n$$

wobei A statistisch verteilt H oder $CH_3$ und n eine Zahl von 5-500, vorzugsweise 8-300, ist,

die Gruppen B gleich oder verschieden sind und Wasserstoff, ein aliphatischer, alicyclischer oder aromatischer Rest mit $C_1$- bis $C_8$-Atomen, vorzugsweise H oder $C_1$- bis $C_4$-Alkyl-, eine Gruppe X–CO– mit X=Wasserstoff, aliphatischer, alicyclischer oder aromatischer Rest mit $C_1$- bis $C_7$-Atomen, vorzugsweise H oder $C_1$- bis $C_3$-Alkyl-, eine Gruppe Y–O–CO– mit Y=aliphatischer, cycloaliphatischer oder aromatischer Rest mit $C_1$- bis $C_7$-Atomen, vorzugsweise $C_1$- bis $C_4$-Alkyl-, sowie

eine Gruppe Z–NH–CO– mit Z=Wasserstoff, aliphatischer, cycloaliphatischer oder aromatischer Rest mit $C_1$- bis $C_7$-Atomen vorzugsweise H oder $C_1$- bis $C_4$-Alkyl- sind,
R ein zweiwertiges Radikal eines Diisocyanates bedeutet und m eine Zahl von 1 bis 30, vorzugsweise 1 bis 15, bedeutet.

Besonders eignen sich als Wirkstoffe für die erfindungsgemässen Mittel wasserlösliche Additionsprodukte aus einem Polyetherdiol und einem Diisocyanat der allgemeinen Formel

$$H{-}[(OCH_2CH_2)_{\bar{n}}{-}O{-}CO{-}NH{-}R{-}NH{-}CO]_{\bar{m}}(OCH_2CH_2)_{\bar{n}}{-}OH$$

entsprechen, in welcher R den bivalenten Rest eines Diisocyanats darstellt und $\bar{m}$ Zahlen von 1 bis 30 und $\bar{n}$ Zahlen von 5 bis 500 bedeuten.

Besonders gut für die Herstellung der erfindungsgemässen Additionsprodukte geeignete Diisocyanate sind Hexamethylendiisocyanat und Isophorondiisocyanat.

In einer besonderen Ausführungsform der Erfindung besitzen die als Wirkstoffe verwendeten Polyadditionsverbindungen ionische Gruppen, wie Carboxyl-, Sulfonat- oder Ammoniumgruppen. Zweckmässigerweise werden diese ionischen Gruppen dadurch in die Polyadditionsverbindungen eingeführt, dass man Diole einsetzt oder miteinsetzt, die die gewünschten Gruppen tragen. So kann beispielsweise zur Herstellung eines ionische Gruppen tragenden Wirkstoffes as Diol der Formel

$$H{-}(O{-}CH_2CH_2)_6O{-}CH_2{-}CH(SO_3Na){-}CH_2{-}CH_2{-}O(CH_2{-}CH_2{-}O)_6H$$

eingesetzt werden.

Beispiele für erfindungsgemäss zu verwendende wasserlösliche Polyadditionsverbindungen sind

$$H{-}[(OCH_2CH_2)_{23}{-}O{-}CONH{-}(CH_2)_6{-}NHCO]_4{-}(OCH_2CH_2)_{23}{-}OH \quad (I)$$

$$H{-}[(OCH_2CH_2)_{7}{-}O{-}CONH{-}(CH_2)_6{-}NHCO]_4{-}(OCH_2CH_2)_{7}{-}OH \quad (II)$$

$$H{-}[(OCH_2CH_2)_{15}{-}O{-}CONH{-}(CH_2)_6{-}NHCO]_{10}{-}(OCH_2CH_2)_{15}{-}OH \quad (III)$$

$$H{-}[(OCH_2CH_2)_{35}{-}O{-}CONH{-}(CH_2)_6{-}NHCO]_5{-}(OCH_2CH_2)_{35}{-}OH \quad (IV)$$

$$H{-}[(OCH_2CH_2)_{68}{-}O{-}CONH{-}(CH_2)_6{-}NHCO]_2{-}(OCH_2CH_2)_{68}{-}OH \quad (V)$$

$$H{-}[(OCH_2CH_2)_{68}{-}O{-}CONH{-}(CH_2)_6{-}NHCO]_4{-}(OCH_2CH_2)_{68}{-}OH \quad (VI)$$

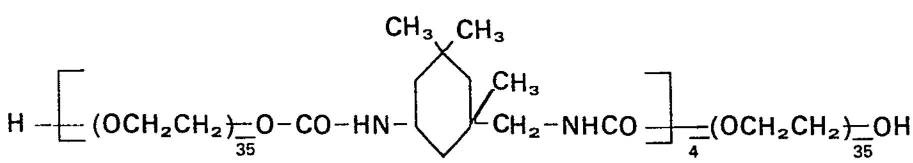

(VII)

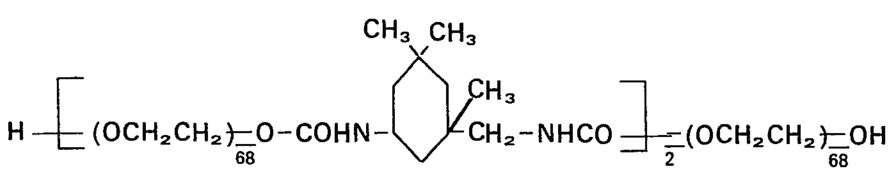

(VIII)

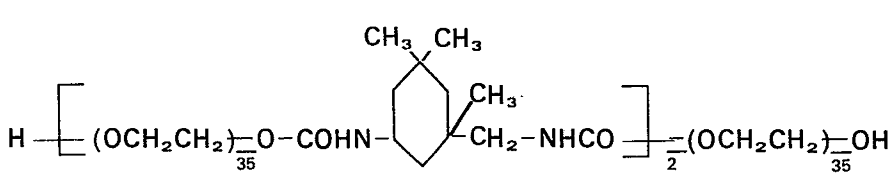

(IX)

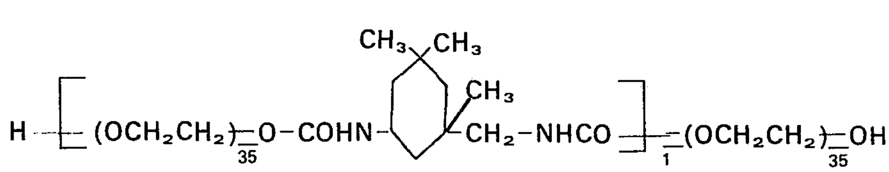

(X)

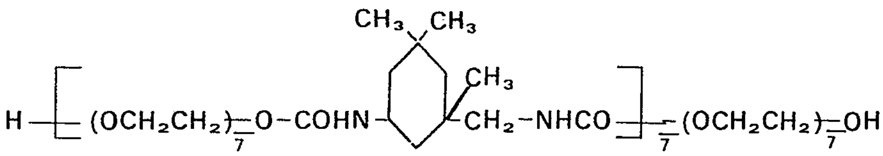

(XI)

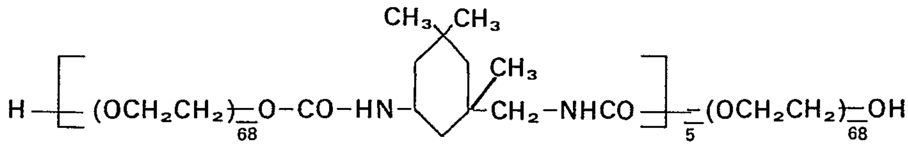

(XII)

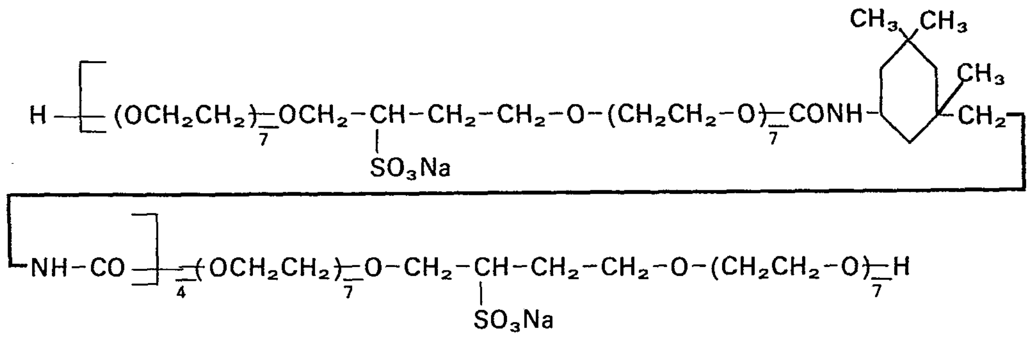

(XIII)

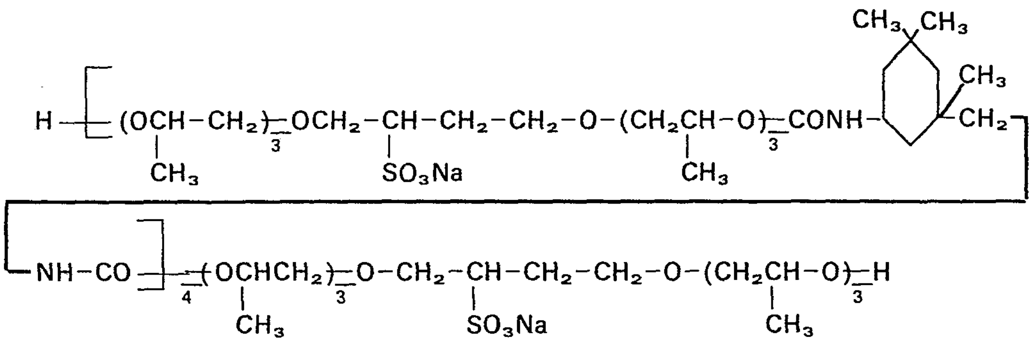

(XIV)

Die wasserlöslichen Polyadditionsprodukte, die die erfindungsgemässen Wirkstoffe darstellen, werden nach an sich bekannten Verfahren hergestellt. Man erhält sie durch Umsetzung von Polyetherglykolen verschiedener Kettenlänge, die gegebenenfalls ionische Gruppen, wie Sulfonat-, Carboxylat- oder Ammoniumgruppen enthalten können, mit Diisocyanaten, insbesondere mit Hexamethylendiisocyanat oder Isophorondiisocyanat.

Diese Umsetzungen erfolgen in einem inerten Lösungsmittel, wie vorzugsweise Chloroform oder Methylenchlorid. Das Mengenverhaltnis von Diol und Diisocyanat wird so gewählt, dass gezielt mehr oder weniger lange Molekülketten resultieren. Immer wird das Diisocyanat im Unterschuss eingesetzt, damit Polymere mit OH-Endgruppen entstehen. Reaktionstemperatur ist Raumtemperatur bis Siedetemperatur des Lösungsmittels. Die Reaktionszeit beträgt 2-8 h.

Die Hydroxylengruppen der Oligo- bzw. Polyurethane können nach bekannten Methoden in Ethergruppen, Carbonsäureestergruppen, Kohlensäureestergruppen oder Urethangruppen überführt werden.

Als Alkylierungsmittel zur Veretherung der Hydroxylgruppen seien beispielsweise Dialkylsulfate, Alkylhalogenide sowie Ester der p-Toluolsulfonsäure genannt (Organikum, Organisch-chemisches Grundpraktikum, 6. Aufl., Berlin 1967, S. 185 ff.). Die Einführung von Carbonsäureestergruppen kann beispielsweise durch Einwirkung von Säureanhydriden in Gegenwart eines sauren Katalysators oder durch Umsetzung mit Säurechloriden in Gegenwart eines tertiären Amins erfolgen. Kohlensäureestergruppen können durch Umsetzung mit Chlorameisensäureestern in Gegenwart inerter Basen hergestellt werden.

Setzt man bei der Polyurethanherstellung Gemische von Diisocyanaten und Monoisocyanaten ein, so erhält man Produkte mit Urethanendgruppen.

Nach Beendigung der Reaktion wird das Lösungsmittel vorzugsweise durch Abdestillieren im Vakuum entfernt. Es werden flüssige, viskose bis feste Substanzen erhalten, die durch ihren Schmelzpunkt bzw. ihren Brechungsindex charakterisiert werden können, und welche in Wasser, oder in physiologischer Kochsalzlösung, gegebenenfalls unter leichter Trübung zu mindestens 0,5 gew.-%igen Lösungen löslich sind.

Die erfindungsgemässen Mittel haben bei äusserst niedriger Toxizität eine starke antitumorale Wirkung gegen tierische und menschliche Tumoren und sollen daher zur Bekämpfung von durch Tumoren verursachten Erkrankungen verwendet werden.

Die erfindungsgemässen Mittel werden hergestellt, indem man die Wirkstoffe in physiologischer Kochsalzlösung löst. Sie können aber auch in Tabletten, Kapseln, Säfte oder andere Zubereitungen für die orale Applikation eingearbeitet werden.

Die dabei erhaltenen Zubereitungen werden intraperitoneal, intravenös, intramuskulär oder oral appliziert. Der therapeutische Dosisbereich reicht von 0,5 bis 500 mg/kg, vorzugsweise 5 bis 250 mg/kg. Die ungewöhnliche Breite dieses Bereiches beruht auf der ungewöhnlichen Ungiftigkeit der Wirkstoffe.

Die in der Tabelle 1 aufgeführten Substanzen wurden in zahlreichen Versuchen unter verschiedenen Testbedingungen an Mäusesarkom MCS 4 sowie am Carcinom EO 771 auf die Induktion von Tumorhemmwirkungen geprüft.

Die Methodik der Untersuchungen an diesen beiden experimentellen Tumoren sind in den nachstehenden Versuchsbeschreibungen a und b enthalten.

*Versuchsbeschreibung a*

*Tumorteste am Carcinom EO 771 auf C 57 BL/6-Mäuse*

Tierart: C 57 BL/6-Mäuse, Inzucht (SPF)

Verfahren: *Stammhaltung:* Am 14.-20. Tag nach letzter Transplantation subkutane Verimpfung einer Zellsuspension des Carcinoms EO 771 in 0,5 ml phosphatgepufferter 0,9%iger NaCl-Lösung auf C 57 BL/6-Mäuse.

*Vorbereitung von Screening-Testen:* Gleiches Verfahren wie bei Stammhaltung des Tumors, jedoch subkutane Verimpfung einer Suspension von $5 \times 10^4$ Tumorzellen in 0,5 ml phosphatgepufferter 0,9%iger NaCl-Lösung.

*Behandlung:* Einmalige, intramuskuläre Injektion der geforderten Präparatlösungen am 6. Tag vor bzw. am 2. Tag nach der Tumortransplantation.

*Versuchsdauer:* 18-22 d nach der Tumortransplantation. Dann Abtötung der Tiere, Präparation und Wägung der subkutanen Tumoren.

*Auswertungsparameter:* Hemmung des Tumorwachstums durch Bestimmung des durchschnittlichen Tumorgewichts von Kontrollen und behandelten Tiergruppen sowie Errechnung des Tumorgewichts (TG)-Index nach der Formel

$$\text{TG-Index} = \frac{\varnothing\ \text{Tumorgewicht der behandelten Tiergruppen}}{\varnothing\ \text{Tumorgewicht der Kontrollgruppe}}$$

*Beurteilung der Testergebnisse:*

TG-Index 0,8 - 0,6 = Wirkungsandeutung;
0,6 - 0,4 = deutliche Wirkung;
<0,4 = gute Wirkung.

*Versuchsbeschreibung b*

*Tumorteste am Sarkom MCS 4 auf C 57 BL/6-Mäuse*

*Stammhaltung:* 10-14 d nach letzter Transplantation subkutane Verimpfung einer Tumorsuspension des Sarkoms MCS 4 in 0,5 ml einer phosphatgepufferten 0,9%igen NaCl-Lösung auf C 57 BL/6-Mäuse.

*Vorbereitung von Screening-Testen:* Gleiches Verfahren wie bei Stammhaltung des Tumors, jedoch subkutane Verimpfung einer Suspension von $2 \times 10^5$ Tumorzellen in 0,5 ml einer phosphatgepufferten 0,9%igen NaCl-Lösung.

*Behandlung:* Einmalige, intravenöse Injektion der geforderten Präparatlösung am 2. Tag vor bzw. am 2. Tag nach der Tumortransplantation.

*Versuchsdauer:* 18-22 d nach der Tumortransplantation. Dann Abtötung der Tiere, Präparation und Wägung der Tumoren.

*Auswertung und Beurteilung der Ergebnisse* erfolgen analog wie in der Versuchsbeschreibung a.

*Gesamtbeurteilung der Ergebnisse in Tabellen 1 und 2*

Die Tumorgewichts-Indizes der aufgeführten Präparate in den Tabellen 1 und 2 lassen erkennen, dass die Substanzen sowohl am Sarkom MCS 4 als auch am Carcinom EO 771 bei verschiedenen Dosierungen und Applikationswegen sowie an verschiedenen Behandlungstagen deutliche Tumorhemmwirkungen zu induzieren vermögen.

*Tabelle 1*

Prüfergebnisse am Sarkom MCS 4

| Verbindung Nr. | Dosis (mg/kg) | Applikation (1×) | Behandlungstag* | Tumorgewichtsindex |
|---|---|---|---|---|
| XIV (Bsp. 14) | 0,5 | i.v. | −2 | 0,11 |
| | 5 | i.v. | +2 | 0,16 |

*Tabelle 2*

Prüfergebnisse am Sarkom EO 771

| Verbindung Nr. | Dosis (mg/kg) | Applikation (1 ×) | Behandlungstag* | Tumorgewichtsindex |
|---|---|---|---|---|
| I (Bsp. 1) | 2,5 | i.m. | −6 | 0,26 |
| | 10 | i.m. | +2 | 0,27 |
| VII (Bsp. 7) | 2,5 | i.m. | −6 | 0,22 |
| | 10 | i.m. | +2 | 0,23 |
| VIII (Bsp. 10) | 10 | i.m. | −6 | 0,39 |
| | 10 | i.m. | +2 | 0,29 |
| IX (Bsp. 12) | 50 | i.m. | −6 | 0,37 |
| | 50 | i.m. | +2 | 0,42 |

* Behandlungstag: −6 = 6 d vor Tumortransplantation
+2 = 2 d nach Tumortransplantation

*Beispiel 1:*

Wasserlösliches Polyurethan der Formel I

$H\{(OCH_2CH_2)_{23}-O-CONH-(CH_2)_6-NHCO\}_4-(OCH_2CH_2)_{23}-OH$

In einer geschlossenen Dreihalskolben-Rührapparatur, die mit Thermometer, Tropftrichter und Rückflusskühler ausgerüstet ist, wird eine Lösung von 50,5 Gew.-Teilen Polyglykol mit einem mittleren Molekulargewicht von 1000 in 50 Vol.-Teilen Chloroform vorgelegt. Unter ständigem Rühren wird eine Lösung von 6,7 Gew.-Teilen Hexamethylendiisocyanat in 50 Vol.-Teilen Chloroform zugetropft. Nach 2stündigem Nachrühren bei 50°C wird das Lösungsmittel am Rotationsverdampfer abgedampft. Man erhält eine kristalline wasserlösliche Substanz. Schmelzpunkt: Fp.=42°C.

*Beispiel 2:*

Polyurethan der Formel II

$H\{(OCH_2CH_2)_7-O-CONH-(CH_2)_6-NHCO\}_4-(OCH_2CH_2)_7-OH$

Wie in Beispiel 1 beschrieben, werden 15 Gew.-Teile Polyglykol mit dem mittleren Molekulargewicht 300, gelöst in 50 Vol.-Teilen Chloroform, mit einer Lösung von 6,7 Gew.-Teilen Hexamethylendiisocyanat in 50 Vol.-Teilen Chloroform versetzt. Man lässt durch 2stündiges Rühren bei 50°C ausreagieren und zieht schliesslich das Lösungsmittel im Rotationsverdampfer ab. Es resultiert eine viskose, wasserlösliche Flüssigkeit mit einem Brechungsindex $n_D^{20}=1,4876$.

*Beispiel 3:*

Polyurethan der Formel

$H\{(OCH_2CH_2)_{8.5}-O-CO-NH-(CH_2)_6-NH-CO\}_4-(OCH_2CH_2)_{8.5}-OH$

Analog Beispiel 1 werden 20 Gew.-Teile Polyglykol mit einem mittleren Molekulargewicht von ca. 400 mit 6,7 Gew.-Teilen Hexamethylendiisocyanat in Chloroform umgesetzt. Man erhält eine gut wasserlösliche, viskose Flüssigkeit mit einem Brechungsindex $n_D^{20}=1,4850$.

*Beispiel 4:*

Polyurethan der Formel VI

$H\{(OCH_2CH_2)_{68}-O-CONH-(CH_2)_6-NHCO\}_4-(OCH_2CH_2)_{68}-OH$

In Methylenchlorid werden, wie in Beispiel 1 beschrieben, 150 Gew.-Teile des Polyglykols mit dem mittleren Molekulargewicht 3000 mit 6,7 Gew.-Teilen Hexamethylendiisocyanat umgesetzt. Man erhält nach Abdampfen des Lösungsmittels eine kristalline Substanz, die unter leichter Trübung in Wasser löslich ist. Schmelzpunkt: Fp.=53°C.

*Beispiel 5:*

Polyurethan der Formel

$H\{(OCH_2CH_2)_{35}-O-CO-NH-(CH_2)_6-NH-CO\}_4-(OCH_2CH_2)_{35}-OH$

Durch Umsetzung von 77,5 Gew.-Teilen des Polyglykols mit dem mittleren Molekulargewicht 1550 und 6,7 Gew.-Teilen Hexamethylendiisocyanat erhält man eine in Wasser lösliche kristalline Substanz mit dem Schmelzpunkt: Fp.=45°C.

*Beispiel 6:*

Polyurethan der Formel

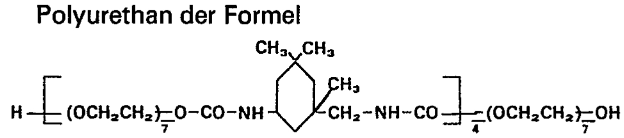

Analog Beispiel 1 wird eine Lösung von 15 Gew.-Teilen eines Polyglykols mit dem mittleren Molekulargewicht 300 in 50 Vol.-Teilen Chloroform mit einer Lösung von 8,9 Gew.-Teilen Isophorondiisocyanat in 50 Vol.-Teilen Chloroform versetzt. Nach 2stündigem Nachrühren bei 50°C wird das Chloroform im Rotationsverdampfer abgedampft. Man erhält eine leicht viskose Flüssigkeit, die sehr leicht in Wasser löslich ist. Brechungsindex $n_D^{20}=1,4952$.

*Beispiel 7:*

Polyurethan der Formel VII

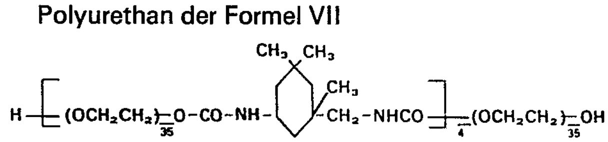

Analog Beispiel 1 werden 77,5 Gew.-Teile des Polyglykols mit dem mittleren Molekulargewicht 1550 mit 8,9 Gew.-Teilen Isophorondiisocyanat umgesetzt. Das resultierende Polyurethan ist eine kristalline Substanz, die bis zu 10% gut in Wasser löslich ist. Schmelzpunkt: Fp.=56°C.

*Beispiel 8:*

Polyurethan der Formel

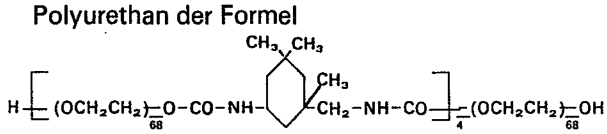

Aus 150 Gew.-Teilen Polyglykol mit dem mittleren Molekulargewicht 3000 und 8,9 Gew.-Teilen Isophorondiisocyanat erhält man ein Polyurethan, das unter leichter Trübung in Wasser löslich ist und einen Schmelzpunkt: Fp.=47°C besitzt.

*Beispiel 9:*

Polyurethan der Formel

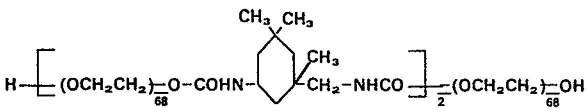

Wie in Beispiel 1 beschrieben werden 102 Gew.-Teile des Polyglykols mit dem mittleren Molekulargewicht 3000 mit 5 Gew.-Teilen Isophorondiisocyanat umgesetzt. Man erhält eine kristalline Substanz, die in Wasser sehr gut löslich ist, mit einem Schmelzpunkt: Fp.=54°C.

*Beispiel 10:*

Polyurethan der Formel XI

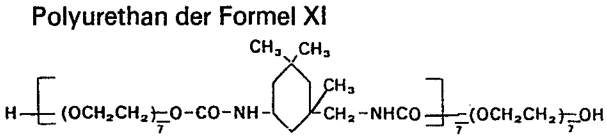

Durch Umsetzung von 24 Gew.-Teilen des Polyglykols mit dem mittleren Molekulargewicht 300 und 15,5 Gew.-Teilen Isophorondiisocyanat in Chloroform erhält man nach Abdampfen des Lösungsmittels im Rotationsverdampfer eine gut in Wasser lösliche, leicht viskose Flüssigkeit mit dem Brechungsindex $n_D^{20}=1{,}4968$.

*Beispiel 11:*

Polyurethan der Formel

$$H\{(OCH_2CH_2)_{68}O-CO-NH-(CH_2)_6-NH-CO\}_{2}(OCH_2CH_2)_{68}OH$$

Nach Umsetzung von 120 Gew.-Teilen Polyglykol mit dem mittleren Molekulargewicht 3000 mit 4,5 Gew.-Teilen Hexamethylendiisocyanat in Methylenchlorid erhält man nach Abdampfen des Lösungsmittels im Vakuum eine gut in Wasser lösliche kristalline Substanz mit einem Schmelzpunkt: Fp.=55°C.

*Beispiel 12:*

Polyurethan der Formel IX

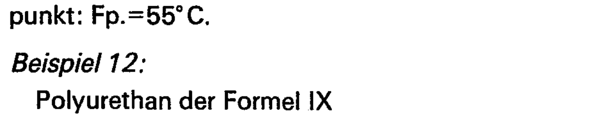

93 Gew.-Teile des Polyglykols mit dem mittleren Molekulargewicht 1550 und 8,9 Gew.-Teile Isophorondiisocyanat werden in Chloroform miteinander umgesetzt. Nach Abdampfen des Lösungsmittels im Vakuum erhält man eine kristalline Substanz, die leicht in Wasser löslich ist. Schmelzpunkt: Fp.=49°C.

*Beispiel 13:*

Polyurethan der Formel X

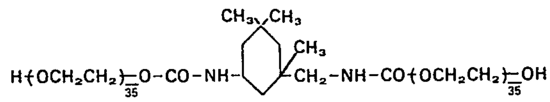

124 Gew.-Teile des Polyglykols mit dem mittleren Molekulargewicht 1550 und 8,9 Gew.-Teile Isophorondiisocyanat werden in Chloroform miteinander umgesetzt. Nach Abdampfen des Lösungsmittels erhält man eine kristalline Substanz, die sich leicht in Wasser lösen lässt. Schmelzpunkt: Fp.=56°C.

*Beispiel 14:*

Polyurethan der Formel XIV

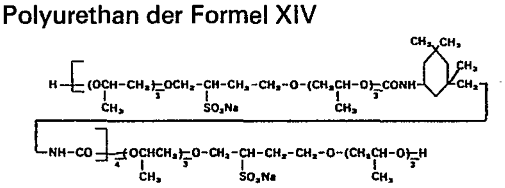

254 Gew.-Teile des Sulfonatdiols der Formel

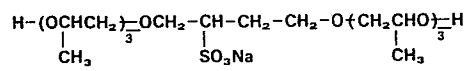

werden in 500 Vol.-Teilen Toluol gelöst und auf Rückflusstemperatur erwärmt. Dann werden 83 Gew.-Teile Isophorondiisocyanat langsam zugegeben. Nach 2stündigem Nachrühren wird das Toluol im Vakuum abdestilliert. Man erhält eine leicht in Wasser lösliche kristalline Substanz.

**Patentansprüche**

1. Antitumoral wirkende Mittel, gekennzeichnet durch einen Gehalt an mindestens einem wasserlöslichen Additionsprodukt aus einem Polyetherdiol und einem Diisocyanat.

2. Antitumoral wirkende Mittel nach Anspruch 1, gekennzeichnet durch einen Gehalt an mindestens einem wasserlöslichen Polyadditionsprodukt aus einem Polyethylenglykol und einem Diisocyanat der allgemeinen Formel

$$H\{(OCH_2CH_2)_{\bar{n}}O-CO-NH-R-NH-CO\}_{\bar{m}}(OCH_2CH_2)_{\bar{n}}OH$$

in welcher R den bivalenten Rest eines Diisocyanats darstellt und $\bar{m}$ Zahlen von 1 bis 30 und $\bar{n}$ Zahlen von 5 bis 500 bedeuten.

3. Antitumoral wirkende Mittel nach Anspruch 2, dadurch gekennzeichnet, dass sich der bivalente Rest des Diisocyanats vom Hexamethylendiisocyanat herleitet.

4. Antitumoral wirkende Mittel nach Anspruch 2, dadurch gekennzeichnet, dass sich der bivalente Rest des Diisocyanats vom Isophorondiisocyanat herleitet.

5. Antitumoral wirkende Mittel nach Anspruch 1, dadurch gekennzeichnet, dass das wasserlösliche Additionsprodukt aus einem Polyetherdiol und einem Diisocyanat ionische Gruppen trägt.

6. Antitumoral wirkende Mittel nach Anspruch 5, dadurch gekennzeichnet, dass das wasserlösliche Additionsprodukt aus einem Polyetherdiol, das Sulfonatgruppen aufweist, und einem Diisocyanat hergestellt wird.

7. Antitumoral wirkende Mittel nach den Ansprüchen 5 und 6, dadurch gekennzeichnet, dass zur Herstellung des wasserlöslichen, ionische Gruppen tragenden Additionsprodukts als Diisocyanat das Hexamethylendiisocyanat verwendet wird.

8. Antitumoral wirkende Mittel nach den Ansprüchen 5 und 6, dadurch gekennzeichnet, dass zur Herstellung des wasserlöslichen, ionische Gruppen tragenden Additionsprodukts als Diisocyanat das Isophorondiisocyanat verwendet wird.

9. Verfahren zur Herstellung eines antitumoral wirkenden Mittels, dadurch gekennzeichnet, dass man mindestens ein wasserlösliches Additionsprodukt aus einem Polyetherdiol und einem Isocyanat nach den Ansprüchen 1 bis 8 in einer physiologischen Kochsalzlösung löst, oder in Zubereitungen für die orale Applikation einarbeitet.

## Claims

1. Agents having a tumour-inhibiting action, characterised in that they contain at least one water-soluble addition product of a polyether-diol and a diisocyanate.

2. Agents having a tumour-inhibiting action, according to Claim 1, characterised in that they contain at least one water-soluble polyaddition product of a polyethylene glycol and a diisocyanate, of the general formula

$$H[(OCH_2CH_2)_{\overline{n}}O-CO-NH-R-NH-CO]_{\overline{m}}(OCH_2CH_2)_{\overline{n}}OH$$

in which

R represents the bivalent radical of a diisocyanate,

$\overline{m}$ denotes a number from 1 to 30, and

$\overline{n}$ denotes a number from 5 to 500.

3. Agents having a tumour-inhibiting action, according to Claim 2, characterised in that the bivalent radical of the diisocyanate is derived from hexamethylene diisocyanate.

4. Agents having a tumour-inhibiting action, according to Claim 2, characterised in that the bivalent radical of the diisocyanate is derived from isophorone diisocyanate.

5. Agents having a tumour-inhibiting action, according to Claim 1, characterised in that the water-soluble addition product of a polyether-diol and a diisocyanate carries ionic groups.

6. Agents having a tumour-inhibiting action, according to Claim 5, characterised in that the water-soluble addition product is prepared from a polyether-diol, which contains sulphonate groups, and a diisocyanate.

7. Agents having a tumour-inhibiting action, according to Claims 5 and 6, characterised in that hexamethylene diisocyanate is used as the diisocyanate for the preparation of the water-soluble addition product carrying ionic goups.

8. Agents having a tumour-inhibiting action, according to Claims 5 and 6, characterised in that isophorone diisocyanate is used as the diisocyanate for the preparation of the water-soluble addition product carrying ionic groups.

9. Process for the preparation of an agent having a tumour-inhibiting action, characterised in that at least one water-soluble addition product of a polyether-diol and an isocyanate, according to Claims 1 to 8, is dissolved in a physiological sodium chloride solution or is incorporated in preparations for oral administration.

## Revendications

1. Produits à action antitumeur, caractérisés en ce qu'ils contiennent au moins un produit d'addition hydrosoluble d'un polyesterdiol et d'un diisocyanate.

2. Produits à action antitumeur selon la revendication 1, caractérisés en ce qu'ils contiennent au moins un produit de polyaddition hydrosoluble d'un polyéthylèneglycol et d'un diisocyanate de formule générale:

$$H[(OCH_2CH_2)_{\overline{n}}O-CO-NH-R-NH-CO]_{\overline{m}}(OCH_2CH_2)_{\overline{n}}OH$$

dans laquelle R représente le reste divalent d'un diisocyanate et $\overline{m}$ représente des nombres de 1 à 30 et $\overline{n}$ des nombres de 5 à 500.

3. Produits à action antitumeur selon la revendication 2, caractérisés en ce que le reste divalent du diisocyanate dérive du diisocyanate d'hexaméthylène.

4. Produits à action antitumeur selon la revendication 2, caractérisés en ce que le reste divalent du diisocyanate dérive du diisocyanate d'isophorone.

5. Produits à action antitumeur selon la revendication 1, caractérisés en ce que le produit d'addition hydrosoluble d'un polyétherdiol et d'un diisocyanate porte des groupes ioniques.

6. Produits à action antitumeur selon la revendication 5, caractérisés en ce que le produit d'addition hydrosoluble est fabriqué à partir d'un polyétherdiol présentant des groupes sulfonates et d'un diisocyanate.

7. Produits à action antitumeur selon les revendications 5 et 6, caractérisés en ce que l'on utilise comme diisocyanate le diisocyanate d'hexaméthylène pour la fabrication du produit d'addition hydrosoluble à groupes ioniques.

8. Produits à action antitumeur selon les revendications 5 et 6, caractérisés en ce que l'on utilise comme diisocyanate le diisocyanate d'isophorone pour la fabrication du produit d'addition hydrosoluble à groupes ioniques.

9. Procédé pour la fabrication d'un produit à action antitumeur, caractérisé en ce que l'on dissout au moins un produit d'addition hydrosoluble d'un polyétherdiol et d'un isocyanate selon les revendications 1 à 8 dans une solution physiologique de chlorure de sodium, ou bien on l'incorpore dans des préparations pour l'administration orale.